# EUROPEAN PATENT APPLICATION

(11) **EP 2 489 355 A1**
(43) Date of publication of application: **22.08.2012**
(21) Application number: 12167804.9
(22) Date of filing: 03.08.2006
(51) Int. Cl.: A61K 31/728, A61K 33/40, A61P 17/02, A61K 45/06, A61K 36/00

(54) **Wound-healing composition**

(30) Priority: 11.08.2005 IT MI20051569
(62) Divisional of application: 06776586.7
(71) Applicant: BMG PHARMA s.r.l., 20124 Milano (IT)
(72) Inventor: Braguti, Gianluca, 23900 LECCO (IT); Delsignore, Giorgio, 23842 BOSISIO PARINI (LC) (IT); Borgonovo, Margherita, 23842 BOSISIO PARINI (LC) (IT); Introini, Carlo, 23842 BOSISIO PARINI (LC) (IT)
(74) Representative: Minoja, Fabrizio

(57) **Abstract**

Disclosed are topical formulations containing hyaluronic acid, hydrogen peroxide or other antiseptic agents and optionally adenosine, aminoacids and plant extracts.

## Description

This invention relates to compositions including hyaluronic acid and hydrogen peroxide (or other disinfectant substances) and possibly other accelerators of the wound-healing process such as aminoacids, adenosine and plant extracts.

### BACKGROUND TO THE INVENTION

The wound-healing process is a complex phenomenon which comprises several stages (inflammation, cell proliferation and tissue reconstruction) involving the epidermis, dermis, extracellular matrix, plasma protein and growth factors.

The wound-healing process does not begin if there is an infection secondary to the penetration of pathogenic agents through the damaged skin. It is therefore essential to use molecules with an antimicrobial activity.

Hydrogen peroxide is a potent oxidising agent with antibacterial, antiviral and fungicidal power. Hydrogen peroxide is also a metabolite of the immune system, produced by the neutrophil granulocytes as the first response to attack by toxins, parasites, bacteria, viruses and yeasts.

Other oxidising agents and/or sources of oxygen, such as sodium perborate, sodium percarbonate, carbamyl peroxide (urea peroxide), potassium permanganate, etc., can be used to reduce the microbe count.

Other known disinfectant agents include quaternary ammonium compounds (benzalkonium chloride, benzethonium chloride, chlorhexidine digluconate, polymeric biguanide hydrochloride, etc.), phenol derivatives (dichlorobenzyl alcohol, benzyl chlorophenol, chloromethyl phenol, orthophenyl phenol, etc.), formaldehyde releasers (glutaraldehyde, imidazolidinyl urea, etc.), silver and its salts, mercury derivatives (thiomersal, phenylmercury acetate, etc.), triclosan, bronopol, etc..

Examples of wound-healing accelerators include hyaluronic acid, aminoacids and plant extracts.

Hyaluronic acid is a polysaccharide of the extracellular matrix secreted by the fibroblasts (Arderson A; Professional Nurse 2001;17:232-235). Hyaluronic acid plays a crucial part in the tissue repair mechanisms due to its physico-chemical (hygroscopic, rheological and viscoelastic) and biological properties.

Foetal tissues are particularly rich in hyaluronic acid, and are regenerated without scarring after lesions (W.Y. John, G Abantangelo; Wound repair and regeneration 1999;7:79). Hyaluronic acid is also involved in all stages of extracellular tissue repair: at the inflammatory, granulation, re-epithelialisation and wound-healing stages.

Hyaluronic acid-based medications have proved more effective than growth factors in promoting wound-healing (Hu M; J. Biomed. Mater. Res. Appl. Biomater. 2003 15;67(1):586-92).

Examples of aminoacids which promote the wound-healing process are glycine, gamma-aminobutyric acid and arginine.

Glycine is an aminoacid which is a basic component of important biological molecules, including collagen. It is a key substance in many metabolic reactions, has anti-inflammatory, cytoprotective and immunomodulating properties, and is the main neurotransmitter with inhibitory activity (Gundersen Y; Tidsskr Nor Laegeforen. 2004 Mar 18;124(6):773-5).

It has been discovered in recent years that there are glycine-dependent chlorine channels in the endothelial cells (Denda M.J Invest Dermatol. 2003 Aug; 121(2):362-7) and epithelial cells, and that their stimulation accelerates the restoration of the endothelial and cutaneous barrier (Yamashina S. Nutr. Cancer 2001 40(2):197-204).

Gamma-aminobutyric acid similarly accelerates the restoration of the cutaneous barrier (Denda M.; J. Invest. Dermatol. 2002 Nov; 119(5):1041-7).

In elderly skin, excessive hyperpolarisation of the membranes prevents normal reconstitution of the epithelia. In these cases, glycine and gamma-aminobutyric acid, by activating the chlorine channels, restore the normal membrane potential which completes the wound-healing process.

Arginine, the aminoacid precursor of nitric oxide (NO), is not only an important macrophagic mediator of the inflammatory response but also an activator of collagen synthesis at fibroblast level (Witte M.B.; Am. J. Surg. 2002 183(4):406-12).

Arginine is also involved in the synthesis of polyamines, which are essential in all DNA duplication processes. The arginases convert arginine into ornithine, the first step in polyamine synthesis. In order for the wound-healing process to take place correctly, there must be a balance between NO and polyamine synthesis.

In diabetics, and elderly people in general, there is accentuated arginase activity, whereas nitric oxide expression is deficient (Kampfer H.; J. Invest. Dermatol., 2004.121(6):1544-51; Sell R.D.; J.Biol.Chem., 2003.279 (52): 54173-84). Some studies have already established the efficacy of arginine in healing diabetic ulcers (Arana V. Biomed. Pharmacother. 58(10):588-97).

Finally, numerous extracts of plants such as Ficus bengalensis, Cynodon dactylon, Symplocos racemosa, Rubia cordifolia, Pterocarpus santalinus, Ficus racemosa, Glycyrrhiza glabra, Berberis ristata, Curcuma longa, Dentella asiatica, Euphorbia nerifolia and Aloe vera promote wound healing (Biswas et al. Int. J. Low Extrem. Wounds 2003 2(1):25-29).

### DESCRIPTION OF THE INVENTION

It has now been discovered that the combination of antiseptics such as hydrogen peroxide with hyaluronic acid is particularly effective when applied topically.

The invention therefore provides topical formulations containing hyaluronic acid and antiseptic agents, preferably hydrogen peroxide alone or in admixture with other antiseptics and optionally aminoacids, adenosine and plant extracts.

The compositions according to the invention promote the healing of traumatic and post-operative wounds, post-traumatic and diabetic ulcers, bedsores, folliculitis, impetigo and paronychia in diabetic and non-diabetic patients.

Hyaluronic acid is preferably present in percentages ranging between 0.01 and 5% in weight, and hydrogen peroxide (120 Vol.) between 1 and 15%.

The preferred aminoacids are glycine and/or arginine, each present in percentages ranging between 0.01 and 2% in weight.

Adenosine in percentages from 0.01 to 3% further promotes healing and exerts anti-inflammatory action mediated by leukotrienes and TNF.

Examples of other antiseptic agents are chlorhexidine, urea peroxide, phenylmercury acetate, o-phenyl-phenol and the like.

The compositions according to the invention are preferably in the form of aqueous gels or polyalcohols containing thickening polymers such as cellulose derivatives or acrylic polymers, together with other excipients in conventional use, such as preservatives, perfumes and the like.

The compositions according to the invention are prepared by dissolving hyaluronic acid or one of its salts, and possibly glycine, in deionised water at the first stage. The thickening polymer, if any, and an aqueous solution of any other constituents (antiseptic agent, arginine, plant extracts etc.) are then added to the homogeneous solution obtained. Hydrogen peroxide is then added slowly under continuous agitation until a homogenous dispersion is obtained.

Examples of plant extracts which can be used in the formulations according to the invention are extracts of Ficus bengalensis, Cynodon dactylon, Symplocos racemosa, Rubia cordifolia, Pterocarpus santalinus, Ficus racemosa, Glycyrrhiza glabra, Berberis ristata, Curcuma longa, Dentella asiatica, Euphorbia nerifolia and Aloe vera.

The following examples further illustrate the invention.

### EXAMPLE 1: H₂O₂ GEL (6%) with Carbopol 980

| *Tradename* | *% use* |
|---|---|
| Deionised water | |
| Hyaluronic acid sodium salt | |
| Glycine | |
| Carbopol 980 (carbomer) | |
| 8-hydroxyquinoline sulphate | |
| Hydrogen peroxide 130 VOL | |

| | |
|---|---|
| **pH: 2.90 - 3.50** | |

### Brookfield viscosity mod. DV1+: 7,000 +/- 3,500 cps (impeller S64, 50 RPM)

### EXAMPLE 2: H₂O₂ GEL (6%) with Renex 1500

| *Tradename* | *% use* |
|---|---|
| Deionised water | |
| Hyaluronic acid sodium salt | |
| Glycine | |
| Renex PEG 1500 (PEG-32) | |
| 8-hydroxyquinoline sulphate | |
| Hydrogen peroxide 130 VOL | |

| | |
|---|---|
| **pH: 2.90 - 3.50** | |

### Brookfield viscosity mod. DV1+: 5,000 +/- 3,500 cps (impeller S64, 50 RPM)

### EXAMPLE 3: H₂O₂ GEL (6%) with Natrosol 250 MR

| *Tradename* | *% use* |
|---|---|
| Deionised water | |
| Hyaluronic acid sodium salt | |
| Glycine | |
| Natrosol 250 MR (hydroxyethylcellulose) | |
| 8-hydroxyquinoline sulphate Deionised water | |
| Hydrogen peroxide 130 VOL | |

| | |
|---|---|
| **pH: 2.90 - 3.50** | |

### Brookfield viscosity mod. DV1+: 5,000 +/- 3,500 cps (impeller S64, 50 RPM)

### EXAMPLE 4: H₂O₂ GEL (6%) with Luviskol k90

| *Tradename* | *% use* |
|---|---|
| Deionised water | |
| Hyaluronic acid sodium salt | |
| Glycine | |
| Luviskol k90 (PVP) | |
| 8-hydroxyquinoline sulphate | |
| Hydrogen peroxide 130 VOL | |

| | |
|---|---|
| **pH: 2.90 - 3.50** | |

### Brookfield viscosity mod. DV1+: 4,500 +/- 3,000 cps (impeller S64, 50 RPM)

### EXAMPLE 5: H₂O₂ GEL (6%) with Cellosize pcg10

| *Tradename* | *% use* |
|---|---|
| Deionised water | |
| Hyaluronic acid sodium salt | |
| Glycine | |
| Cellosize PCG10 (hydroxyethylcellulose) | |
| 8-hydroxyquinoline sulphate | |
| Hydrogen peroxide 130 VOL | |

| | |
|---|---|
| **pH: 2.90 - 3.50** | |

### Brookfield viscosity mod. DV1+: 5,000 +/- 3,500 cps (impeller S64, 50 RPM)

### EXAMPLE 6: H₂O₂ GEL (6%) with Keltrol

| *Tradename* | *% use* |
|---|---|
| Deionised water | |
| Hyaluronic acid sodium salt | |
| Glycine | |
| Keltrol FCC-E415 (xanthan gum) | |
| 8-hydroxyquinoline sulphate | |
| Hydrogen peroxide 130 VOL | |

| | |
|---|---|
| **pH: 2.90 - 3.50** | |

### Brookfield viscosity mod. DV1+: 6,000 +/- 3,000 cps (impeller S64, 50 RPM)

### EXAMPLE 7: H₂O₂ GEL (6%): Carbopol 980+Luviskol k90

| *Tradename* | *% use* |
|---|---|
| Deionised water | |
| Hyaluronic acid sodium salt | |
| Glycine | |
| Carbopol 980 (carbomer) Luviskol k90 (PVP) | |
| 8-hydroxyquinoline sulphate | |
| Hydrogen peroxide 130 VOL | |

| | |
|---|---|
| **pH: 2.90 - 3.50** | |

### Brookfield viscosity mod. DV1+: 4,000 +/- 2,000 cps (impeller S64, 50 RPM)

### EXAMPLE 8: H₂O₂GEL (6%): Carbopol 980+Keltrol

| *Tradename* | *% use* |
|---|---|
| Deionised water | |
| Hyaluronic acid sodium salt | |
| Glycine | |
| Keltrol FCC-E415 (xanthan gum) Carbopol 980 (carbomer) | |
| Hydrogen peroxide 130 VOL | |

| | |
|---|---|
| **pH: 2.90 - 3.50** | |

### Brookfield viscosity mod. DV1+: 8,000 +/- 3,500 cps (impeller S64, 50 RPM)

### EXAMPLE 9: H₂O₂ GEL (6%) with Carbopol 980 and arginine

| *Tradename* | *% use* |
|---|---|
| Deionised water | |
| Hyaluronic acid sodium salt | |
| Arginine | |
| Glycine | |
| Carbopol 980 (carbomer) | |
| 8-hydroxyquinoline sulphate | |
| Hydrogen peroxide 130 VOL | |

| | |
|---|---|
| **pH: 2.90 - 3.50** | |

### Brookfield viscosity mod. DV1+: 7,000 +/- 3,500 cps (impeller S64, 50 RPM)

### EXAMPLE 10: CHLORHEXIDINE GEL with Renex 1500

| *Tradename* | *% use* |
|---|---|
| Deionised water | |
| Hyaluronic acid sodium salt | |
| Glycine | |
| Renex PEG 1500 (PEG-32) | |
| Arginine | |
| Chlorhexidine gluconate | |

### EXAMPLE 11: Urea peroxide GEL with Carbopol 980

| *Tradename* | *% use* |
|---|---|
| Deionised water | |
| Glycerin | |
| Hyaluronic acid sodium salt | |
| Glycine | |
| Carbopol 980 (carbomer) | |
| Arginine | |
| Urea peroxide | |

### EXAMPLE 12: Phenylmercury acetate GEL with Renex 1500

| *Tradename* | *% use* |
|---|---|
| Deionised water | |
| Hyaluronic acid sodium salt | |
| Glycine | |
| Renex PEG 1500 (PEG-32) | |
| Arginine | |
| Phenylmercury acetate | |

### EXAMPLE 13: O-phenylphenol GEL with Renex 1500

| *Tradename* | *% use* |
|---|---|
| Deionised water | |
| Hyaluronic acid sodium salt | |
| Glycine | |
| Renex PEG 1500 (PEG-32) | |
| Arginine | |
| O-phenylphenol | |
| Cremophor RH 40 (Peg-40 hydrogenated castor oil) | |

80 patients divided into 4 sub-groups with similar disorders were treated with the formulation described in Example 1:
Group A: 20 women aged 20-65 years suffering from paronychia
Group B: 20 patients: 13 men and women aged 8-50 years with post-operative wounds resulting from DTC in 16 cases, simple wounds in 2 cases, and abrasions in 1 case. The size and depth of the wounds was evaluated;
Group C: 20 patients: 10 women and 10 men aged between 18 and 49 years, suffering from folliculitis, evaluated according to the characteristics of the wound bed;
Group D: 20 patients, 8 men and women aged between 90 and 47 years, suffering from ulcers with a post-traumatic aetiology in 7 cases, venous in 2 cases, and diabetic in 8 cases. The number of lesions, the basic characteristics of the wound bed (cleansed, weeping, current infection), and the size and depth of the wound were evaluated. The patients were treated daily, once or twice a day.

Clinical progress was evaluated as poor, moderate, good or excellent. The observations were made after 5, 10, 15 and 20 days in the case of folliculitis and paronychia. Wounds and ulcers were also evaluated after the 20th day.

Tolerability proved excellent, without any appreciable side effects apart from a negligible, occasional stinging sensation; only one patient dropped out of the trial because of intense stinging.

In folliculitis, the results were judged to be good in 10 cases and excellent in 10 cases.

In paronychia, 16 patients had an excellent result and 4 a good result.

In post-traumatic ulcerative lesions, the results were good in 3 cases and excellent in 2 cases.

In ulcerative lesions with a venous or diabetic aetiology, the formulations according to the invention gave poor results in relation to resolution and wound-healing, although good wound cleansing was maintained.

## Claims

1. Topical formulations containing hyaluronic acid, antiseptic agents and optionally aminoacids, adenosine and plant extracts.

2. Formulations as claimed in claim 1 wherein the antiseptic agents are selected from hydrogen peroxide, chlorhexidine, urea peroxide, phenylmercury acetate and o-phenyl-phenol.

3. Formulations as claimed in claim 2 wherein the antiseptic agent is hydrogen peroxide, optionally in admixture with other antiseptic agents.

4. Formulations as claimed in claims 1-3, also containing glycine and/or arginine.

5. Formulations as claimed in claims 1-4, wherein hyaluronic acid is present in percentages ranging between 0.01 and 5% by weight, and hydrogen peroxide (120 vol.) between 1 and 15%.

6. Formulations as claimed in any of claims 1 to 5, wherein glycine and/or arginine are present in percentages ranging between 0.01 and 2% by weight.

7. Formulations as claimed in any of claims 1 to 6, in the form of an aqueous gel or with polyalcohols containing thickening polymers chosen from among derivatives of cellulose or acrylic polymers.

8. Formulations as claimed in any of claims 1 to 7, also containing plant extracts.

9. Formulations as claimed in any of claims 1 to 8, also containing from 0.01 to 3% by weight of adenosine.

10. Formulations as claimed in any of claims 1 to 9, also containing from 0.01 to 2% by weight of gamma-hydroxy-butyric acid.

11. Formulations as claimed in any of claims 1 to 10, also containing from 0.01 to 2 % by weight of glycine or gamma-hydroxy-butyric acid, from 0.01 to 2% by weight of arginine and from 0.01 to 3% by weight of adenosine.

12. Use of a combination of hyaluronic acid or its salts and of antiseptics, preferably hydrogen peroxide, optionally in admixture with glycine, arginine, gamma-hydroxy-butyric acid, adenosine and/or plant extracts for the preparation of medicinal products or devices which promote healing of traumatic and post-operative wounds, post-traumatic and diabetic ulcers, bedsores, folliculitis, impetigo and paronychia in both diabetics and non-diabetics.
